# EUROPEAN PATENT SPECIFICATION

(11) **EP 4 051 204 B1**
(45) Date of publication and mention of the grant of the patent: **01.10.2025**
(21) Application number: 20807521.8
(22) Date of filing: 28.10.2020
(51) Int. Cl.: A61G 10/02, A61B 5/16

(54) **METHODS AND SYSTEMS FOR MODIFYING COGNITIVE PERFORMANCE**
VERFAHREN UND SYSTEME ZUR MODIFIZIERUNG DER KOGNITIVEN LEISTUNG
PROCÉDÉS ET SYSTÈMES POUR MODIFIER LA PERFORMANCE COGNITIVE

(30) Priority: 29.10.2019 IL 27026319
(43) Date of publication of application: 07.09.2022
(73) Proprietor: Aviv Scientific Ltd, 5126114 Bnei Brak (IL)
(72) Inventor: PREMINGER, Jonathan, 4624567 Herzliya (IL); HADANNY, Amir, 5502913 Qiryat Ono (IL)
(74) Representative: Zacco GmbH
(86) International application number: PCT/IL2020/051120
(87) International publication number: WO 2021/084531

(56) References cited:
- WO-A1-2016/044317
- KR-A- 20190 098 400
- KR-U- 20080 002 753
- US-A1- 2004 261 796
- DATABASE MEDLINE [online] US NATIONAL LIBRARY OF MEDICINE (NLM), BETHESDA, MD, US; 2017, VADAS DOR ET AL: "Hyperbaric Oxygen Environment Can Enhance Brain Activity and Multitasking Performance.", Database accession no. NLM29021747
- VADAS DOR ET AL: "Hyperbaric Oxygen Environment Can Enhance Brain Activity and Multitasking Performance.", FRONTIERS IN INTEGRATIVE NEUROSCIENCE 2017, vol. 11, 2017, pages 25, ISSN: 1662-5145
- VADAS DOR ET AL: "Hyperbaric Oxygen Environment Can Enhance Brain Activity and Multitasking Performance.", FRONTIERS IN INTEGRATIVE NEUROSCIENCE, vol. 11, 25, 27 September 2017 (2017-09-27), pages 1 - 6, XP002801815, ISSN: 1662-5145, DOI: 10.3389/fnint.2017.00025

## Description

### TECHNOLOGICAL FIELD

The present disclosure concerns methods and systems for improving cognitive performance. More specifically, the disclosure concerns methods and systems for cognitive training under hyperbaric conditions.

### BACKGROUND ART

References considered to be relevant as background to the presently disclosed subject matter are listed below:
[1] Vadas et al., Frontiers in Integrative Neuroscience 2017, 11, 25
[2] Gill et al., J Appl Physiol 2014, 117(4), 406-412
[3] Ergen et al., Clin Neurophysiol 2017, 128(4), 579-588
[4] Yu et al., Clin Neurophysiol 2015, 126(11), 2058-2067
[5] Komiyama et al., Sci Rep 2017, 7(1), 10,000
[6] Su et al., Zhonghua Yu Fang 2016, 50(7), 600-604
[7] Bayer et al., Adv Gerontol 2017, 30(2), 255-261
[8] Malle et al., Aerosp Med Hum Perform 2016, 87(1), 3-12
[9] Goodwin et al., Neuroimage 2009, 47(2), 573-580
[10] Chung et al., Physiol Meas 2007, 28(4), 389-396
[11] Scholey et al., Physiol Behav 1999, 67(5), 783-789
[12] Scholey et al., Psychopharmacology 1998, 140(1), 123-126
[13] Tennstedt et al., J Aging Health 2013, 25(8), 3S-20S
[14] Ball et al., JAMA 2002, 288(18), 2271-2281
[15] Noice et al., J Aging Health 2004, 16(4), 562-585
[16] Neely et al., J Gerontol B 1995, 50(3), 134-140
[17] Unverzagt et al., J Int Neuropsychol Soc 2009, 13(6), 953-960
[18] Willis et al., JAMA 2006, 296(23), 2805-2814
[19] Wolinsky et al., J Gerontol A 2006, 61(12), 1324-1329

Acknowledgement of the above references herein is not to be inferred as meaning that these are in any way relevant to the patentability of the presently disclosed subject matter.

### BACKGROUND

Hyperbaric treatment of various conditions is known for many years, for example to treat effects of decompression sickness or carbon monoxide poisoning, treat arterial gas embolism, encourage skin growth in burn victims, *etc.* Such treatments are typically carried out by enclosing the patient in a chamber, in which controlled increase of pressure can be obtained. In some treatment protocols, the patient is also exposed to oxygen enriched environment during hyperbaric treatment **[1-12]**. Cognitive training in ambient conditions has been shown to improve cognitive function in patients suffering from various cognitive impairments **[13-19].** To date, due to the rigorous constrains imposed onto hyperbaric treatment conditions (typically carried out in narrow, tube-like chambers which leave little room for the patient to move, and/or preventing insertion of electrical equipment thereinto due to the oxygen-rich environment), the effect of additional treatment routes carried out during hyperbaric treatment, such as cognitive training has yet to be investigated.

KR 20190098400 A describes a hyperbaric chamber for applying hyperbaric conditions on a single subject. The hyperbaric chamber is designed to be tilted to different angles for physical rehabilitation of motion-impaired patients, and comprises an internal virtual reality (VR) system to project various images to the subject during change of the angle of the chamber to support the subject's experience of the change in physical angle of the chamber.

KR 2008002753 U discloses a system with a chamber in which two patients can be treated.

Further prior art is known from US 2004/261796 A1, WO 2016/044317 A1, and from the scientific publication of VADAS DOR ET AL: "Hyperbaric Oxygen Environment Can Enhance Brain Activity and Multitasking Performance.", FRONTIERS IN INTEGRATIVE NEUROSCIENCE, vol. 11, 25, 27 September 2017, pages 1-6.

It is an object of the present invention to provide a system, which enables obtaining significant improvement of cognitive performance of a subject.

### GENERAL DESCRIPTION

The object of the invention is satisfied by a system in accordance with the features of claim 1. Preferred embodiments of the invention are described in the dependent claims.

The present disclosure concerns methods and systems for modifying (e.g. improving) cognitive functions by combining hyperbaric exposure at oxygen-rich environment with cognitive training that is carried out within the hyperbaric chamber. Such combined treatment is aimed at modifying cognitive functions with a long-lasting effect, namely aiming to obtain modification and maintaining of such modification after the treatment has been carried out (*i.e.* maintaining the treatment effects on cognitive function obtained by the treatment for at least a period of time after carrying out the treatment).

Thus, provided by an aspect of the present disclosure is a method of modifying cognitive performance of a subject, the method comprising treating said subject by one or more treatment cycles, each treatment cycle comprises exposing said subject to hyperbaric conditions and oxygen rich environment, and carrying out at least one cognitive training task during said exposure.

In some embodiments, combining exposure to hyperbaric conditions and oxygen rich environment with at least one cognitive training task carried out during said exposure results in a synergistic effect on cognitive performance modification.

Within the context of the present disclosure, the term *modifying* (or any lingual variation thereof) is meant to encompass induction of at least one desired effect on or resulting from cognitive performance of the subject. Thus, treating a subject by the methods disclosed herein can induce, enhance, improve, arrest or diminish at least one effect associated with cognitive performance, either by virtue of the treatment itself or by inducing associated mechanisms resulting in cognitive performance modification. The term also means to encompass ameliorating undesired symptoms associated with a cognitive disorder, preventing the manifestation of such symptoms before they occur, slowing down the progression of a cognitive disorder, slowing down the deterioration of symptoms associated with a cognitive disorder, slowing down the irreversible damage caused in progressive chronic stage of a cognitive disorder, delaying the onset of said progressive stage, lessening the severity of a cognitive disorder, improving recovery from brain injury, improving normal cognitive function, or a combination of two or more of the above.

In some embodiments, the method improves or enhances cognitive performance of the subject.

In other embodiments, the method diminishes or arrests at least one symptom associated with at least one impaired cognitive function.

In yet other embodiments, the method increases the compliance of the subject to perform cognitive training and/or to complete a set of said one or more treatment cycles.

The term *cognitive performance* (or *cognitive function* to be used in this disclosure interchangeably) is meant to encompass any mental or intellectual process involving perception, thinking, reasoning, memory, sensation, imagination, *etc.,* that enables a subject to carry out a defined mental task. Exemplary, non-limiting, cognitive functions may be verbal memory, nonverbal memory, immediate memory, delayed memory, information processing speed, attention, executive function, motoric function, visual perception, multi-tasking, visual spatial recognition, verbal expression, navigation, intelligence, working memory, task switching, response inhibition, psychomotor speed, verbal fluency, naming of objects, vocabulary identification, *etc.*

The methods of this disclosure involve exposing a subject to be treated to hyperbaric and oxygen-rich conditions (Hyperbaric Oxygen Treatment - HBOT), during which at least one cognitive task is being carried out by the subject.

*Hyperbaric* conditions refer to exposure of the subject to a pressurized environment, *i.e.* an environment in which a pressure above standard atmospheric pressure resides (>1 atm). Such hyperbaric conditions may be obtained by utilization of a hyperbaric chamber, which is a chamber enabling controlled increase of pressure while a subject resides therein. Such hyperbaric chambers may, for example, be suitable for enclosing a single subject, or suitable for simultaneously enclosing two or more subjects.

By some embodiments, the hyperbaric conditions comprise exposing the subject to a pressure of between about 1.1 absolute atmospheres (ATA) and about 3.2 ATA. In other embodiments, the hyperbaric conditions comprise exposing the subject to a pressure of between about 1.2 ATA and about 3 ATA.

Together with increased pressure, the subject is exposed to an oxygen rich environment, which, in some embodiments, is an environment that comprises between about 22 vol% and 100 vol% oxygen. In other embodiments, the oxygen rich environment can comprise about 25 vol%, 30 vol%, 35 vol%, 40 vol%, 45 vol%, 50 vol%, 55 vol%, 60 vol%, 65 vol%, 70 vol%, 75 vol%, 80 vol%, 85 vol%, 90 vol%, 95 vol%, 98 vol%, or even 100 vol% of oxygen. In some other embodiments, the oxygen-rich environment comprises between about 30 vol% and about 100 vol% oxygen, between about 40 vol% to about 100 vol%, between 50 vol% and 100 vol%, or even between 60 vol% and 100%.

In other embodiments, the oxygen rich environment comprises about 21 vol% oxygen provided at above atmospheric pressure.

The oxygen rich environment can be provided in the entire volume of the hyperbaric chamber, or can be administered individually to each subject within the chamber, *e.g.* via a personal mask, hood, cannula, *etc.*

In some embodiments, the level of oxygen in the oxygen-rich environment is maintained constant throughout the treatment cycle.

In other embodiments, exposure to said oxygen rich environment is carried out intermittently. In such embodiments, exposure to said oxygen rich environment may be carried out during defined time periods, with intervals of administration of lower oxygen levels (*e.g.* 21 vol% oxygen or ambient air) in between said defined time periods. Such intervals can, for example, be of between about 10 seconds and 30 minutes, in between administration of oxygen-rich environment. In some embodiments, the intervals can be of between about 1 and about 10 minutes.

According to some embodiments, each treatment cycle may comprise a total (elapsed) time of exposure to said oxygen rich environment of between about 1 minute and 200 minutes. In other embodiments, said total time of exposure can be between about 10 minutes and 180 minutes, between about 10 minutes and 160 minutes, between about 10 minutes and 140 minutes, or even between about 10 minutes and 120 minutes. In some other embodiments, said total time of exposure can be between about 20 minutes and 200 minutes, between about 30 minutes and 180 minutes, between about 40 minutes and 160 minutes, between about 50 minutes and 140 minutes, or even between about 60 minutes and about 120 minutes.

During exposure to the hyperbaric and rich-oxygen conditions (and at times also during the interval between exposure to oxygen rich environment), at least one cognitive training task is being carried out by the subject. In some embodiments, the at least one cognitive training task can be one or more tasks selected from response time, double decision, sustained attention, divided attention, selective attention, working memory, long term memory, reasoning, auditory processing, verbal memory task, nonverbal memory task, immediate memory task, delayed memory task, information processing speed task, attention task, executive function task, motoric task, visual perception task, multi-tasking assignment, visual spatial task, verbal task, navigational task, intelligence task, working memory task, task switching, response inhibition task, psychomotor speed task, verbal functions task (fluency/naming/vocabulary), and others, as well as combinations thereof. The cognitive training or cognitive task(s) may be carried out by any suitable means, *e.g*. by interaction with an operator, interaction with a dedicated device or tool, carrying out a pre-determined test or assignment, *etc.*

The method is typically carried out for at least 2 treatment cycles, by some embodiments between 5 and 120 cycles. The cycles may be carried out daily (*i.e.* 1 cycle per day). Alternatively, a treatment cycle may be carried out every 2 days, every 3 days, every 4 days, every 5 days, every 6 days, weekly, bi-weekly, *etc.* As will become apparent to the practitioner, the methods disclosed herein can also include any consecutive sequences of treatment cycles, for example a number of daily cycles followed by several days of rest *(e.g.* sequences of 5 daily cycles followed by 2 days of rest). It is also to be understood that the number of cycles may vary, depending on the level of desired cognitive performance modification and/or on the subject's medical/cognitive history or condition.

In some embodiments, the treatment conditions (*i.e.* pressure, vol% oxygen, type of cognitive training task, *etc*.) may be the same in each treatment cycle. In other embodiments, the treatment conditions differ from cycle to cycle. It is also to be understood that, in further embodiments, the treatment conditions may vary depending on the subject's condition and level of cognitive performance modification. For example, several cycles may be carried out with a given set of conditions, followed by several cycles with different set of treatment conditions.

Each treatment cycle may, by some embodiments, be carried out for a period of time of between about 1 and 200 minutes.

The method may further comprise, according to some embodiments, assessing cognitive performance of the subject before said exposure to the treatment conditions to obtain a first cognitive performance score. In further embodiments, the method may further comprise assessing cognitive performance of said subject after said exposure to the treatment conditions to obtain a second cognitive performance score. Comparing the first score and second score (or applying any suitable algorithm onto the first and second scores) may enable obtaining an evaluation/assessment score of the subject to determine subject progress. The assessment of cognitive performance may also be carried out at any time during a treatment cycle. In some embodiments, the evaluation score for a given treatment cycle (or after a sequence of several cycles) may determine the treatment conditions in one or more subsequent treatment cycles, for example as compared to one or more of the baseline score, ongoing score during the treatment cycle, a relative score for similar age group, *etc.*

The term *score* is meant to refer to any relevant measurable or assessable value, *i.e.* quantitative or qualitative, by which cognitive performance of a subject can be evaluated.

The method may further comprise a compression stage before said treatment cycle; compression may be typically carried out at a rate of between about 0.1 m/min and about 2 m/min. The method may also comprise a decompression stage after said treatment cycle, which may be typically carried out at a decompression rate of between about 0.1 m/min and about 2 m/min.

In some embodiments, the at least one treatment cycle may further comprise at least one physical training task carried out under said hyperbaric conditions and oxygen rich environment. Physical training can be any suitable physical training known *per se, e.g.* cycling, walking, powerwalking, running, jumping, weightlifting, hand-cycling, *etc.* It is to be understood that, in the context of the present disclosure, physical training may be utilized in addition to cognitive training in order to obtain modification of one or more cognitive functions.

The subject is typically human, either an adult or pediatric (*pediatric* refers to subjects below 18 years of age). The subject may be healthy, suffer from one or more cognitive impairments or injuries, or having a history of brain injury. In some embodiments, the subject has a history of brain injury, for example stroke, traumatic brain injury (TBI), ischemia, hemorrhagic injury, traumatic injury, anoxia, hypoxia, damage due to metabolic disorder, damage due to toxic exposure, and infection. In other embodiments, the subject suffers from a neurodegenerative disease, dementia, or mild cognitive impairment. In some other embodiments, the subject suffers from aging-related cognitive functional decline. In further embodiments, the subject suffers from cerebral palsy. In yet further embodiments, the subject suffers from abnormal development. According to yet some other embodiments, the subject is a healthy subject. According to further embodiments, the subject is pre-diagnosed or undiagnosed with a disorder. According to yet further embodiments, the subject is a professional athlete (*i.e.* the treatment is tailored to improve the athlete's cognitive and mental performance, rather than his/her physical performance).

The present disclosure also provides a system for operating the methods described herein. Thus, in another aspect, there is provided a system for use in a method of this disclosure.

A further aspect of this disclosure is a system for operating the methods described herein, the system comprising at least one hyperbaric chamber configured to enclose at least one subject and apply hyperbaric conditions thereonto; at least one controllable oxygen administration unit permitting forming an oxygen rich environment within the chamber and/or configured to administer said oxygen rich environment to said subject; and at least one means of cognitive training within the chamber configured to permit a subject to carry out said at least one cognitive training task.

In the context of the present disclosure, the term *hyperbaric chamber* means to encompass a single-place chamber (*i.e.* for occupancy of a single user) or a multi-place chamber (for occupancy of one or more users). The chamber may be unitary or divided into sub-chambers or treatment zones; the conditions applied in a sub-chamber or zone may be the same or different than the conditions in another sub-chamber or zone.

According to some embodiments, the hyperbaric chamber may further comprise at least one compression sub-chamber, at least one training sub-chamber, and at least one decompression sub-chamber. The compression sub-chamber, training sub-chamber and decompression sub-chamber may be linked to one another as to permit the subject to migrate therebetween, *e.g*. the sub-chambers may be integral one with the other.

In some embodiments, the hyperbaric chamber may be configured to enclose a single subject to be treated.

In other embodiments, the hyperbaric chamber may be configured to enclose two or more subjects to be treated simultaneously therein. In such embodiments, the at least one controllable oxygen administration unit may be configured to administer a controllable oxygen rich environment to each of the subjects, and/or may be configured to administer a different oxygen rich environment to each of the subjects.

The at least one means of cognitive training may, according to some embodiments, be configured for permitting each of the subjects to independently carry out at least one cognitive training task. The at least one means of cognitive training may be configured for permitting each of the subjects to carry out a different cognitive training task and/or independently from the other subjects in the chamber.

The system may further comprise at least one means of physical training within the chamber that is configured to permit the subject to carry out at least one physical training task.

In some embodiments, the system may further comprise at least one monitoring unit for monitoring at least one physical parameter of the subject (*e.g*. heart rate, oxygen saturation level, blood pressure, glucose level, various markers and/or electrolytes, *etc*.). The monitored physical parameters may, by some embodiments, be used to determine (at least partially) the conditions in one or more subsequent treatment cycles.

In further embodiments, the system may further comprise at least one controller for operating and controlling the system.

The system may also comprise one or more communication means within the chamber, allowing the treated subject and an operator residing outside the chamber to communicate with one another during the treatment session.

The system may also comprise one or more entertainment and/or interactive means/units, for example music players, TV screens, computer interfaces, *etc.*

As used herein, the term *about* is meant to encompass deviation of ±10% from the specifically mentioned value of a parameter, such as time, pressure, vol%, *etc.*

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features detailed in this disclosure, which are, for clarity, described in the context of separate embodiments, may also be provided in combination in a single embodiment. Conversely, various features which are, for brevity, described in the context of a single embodiment, may also be provided separately or in any suitable subcombination or as suitable in any other described embodiment of this disclosure.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

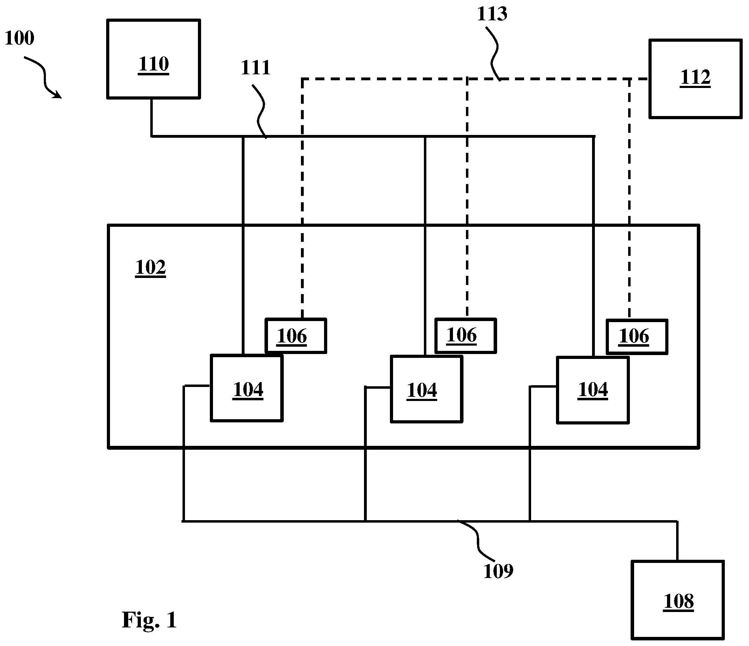

**Fig. 1** is a schematic illustration of a system suitable for carrying out the methods described herein according to an embodiment of this disclosure.

### DETAILED DESCRIPTION OF EMBODIMENTS

In the following description the invention will be illustrated with some details in reference to specific embodiments of a system for carrying out the methods described herein. This illustration is exemplary and non-limiting of the disclosure in its full scope as described.

Referring to **Fig. 1****,** shown is a schematic representation of an exemplary system of this disclosure. System **100** includes a hyperbaric chamber **102,** comprising a plurality of stations **104** (three (3) stations in this specific, non-limiting, example). Each of stations **104** is configured to accommodate a subject to be treated. The stations may, for example, be sitting, standing or laying locations, that once accommodating the subject, permit exposing the subject to hyperbaric conditions and permit the subject to carry out at least one cognitive training task. Each station **104** also comprises means (not shown) for providing the subject with oxygen rich environment (*i.e.* atmosphere with an oxygen concentration higher than 21 vol%); such means may, for example, be oxygen masks, hoods, cannulas, *etc.* The oxygen supplying means are connected via supply line **109** to a controllable oxygen source **108,** thus permitting control of the oxygen level, duration of exposure, oxygen exposure profile/regimen, *etc.* for each of the stations **104,** either collectively or independently of one another. In some configurations, the system further provides an air source **110,** that is linked to each of the stations via supply line **111,** permitting exposure of the subjects to air atmosphere (*i.e.* unpressurized air of about 21 vol% oxygen) in between oxygen-rich atmosphere exposure periods, depending on the treatment regimen required.

Each station **104** is associated with at least one means of cognitive training **106,** permitting the subject to carry out at least one cognitive training task. In the case of electronic/digital training means, the cognitive training means **106** may be linked to a control system **112** via line **113** (which may be wired or wireless). Control system **112** can provide and operate the cognitive training assignments for each subject, as well as collect data from the subject and, at times, also analyze the data received from the subject in order to tailor the cognitive training task to be carried out by the subject.

As noted, although not shown in Fig. 1, the system may comprise further functionalities, such as monitor units, entertainment modules, physical training means, *etc.* Although the system is shown, in this specific example, to include one hyperbaric chamber, it is contemplated within the scope of the present disclosure that the system may comprise a plurality of interlinked chambers or sub-chambers, permitting free transition of subjects therebetween, while maintaining hyperbaric conditions in the system. Each such sub-chamber may, for example, be dedicated to a different type of training or activity to be carried out under hyperbaric and oxygen-rich conditions.

### EXAMPLES

### Example 1: Cognitive training comparative protocol 1

Cognitive training is carried out during hyperbaric, rich oxygen environment exposure for the purpose of cognitive capabilities improvement and/or enhancement. The synergistic effect of cognitive training carried out during hyperbaric oxygen therapy (HBOT) is assessed as compared to cognitive training between HBOT sessions and without HBOT.

Subjects undergoing the treatment are patients with aging related functional decline, age 60-74. Subjects' screening (inclusion criteria) - at least 1 standard deviation (SD) below average in at least one cognitive domain, as measured using a computerized cognitive assessment (for example Neurotrax or any other suitable assessment tool).

Three groups are tested, each group including 10 patients, according to the following grouping:
1. Group 1: HBOT with cognitive training intra-chamber (60 daily cycles, i.e. 1 cycle per day for up to 60 days)
2. Group 2 (control 1): HBOT with cognitive training tasks outside the chamber (60 daily cycles)
3. Group 3 (control 2): Cognitive training tasks only (60 daily cycles)

### Treatment parameters:

- Pressure in chamber: 2 ATA
- Oxygen level: 100%
- Total time: 120 minutes per treatment cycle
- Oxygen net time: 90 minutes per cycle
- Administration of oxygen rich environment by mask
- Total number of cycles: 60
- Number of cycles per week: 5
- Decompression rate: 1 m/min
- Compression rate: 1 m/min
- Air breaks: 5 minutes air break (21% oxygen) between every 20 minutes of rich-oxygen environment administration
- Duration of cognitive training task: 25 minutes per cycle

### Cognitive training

At least one cognitive task selected from (however not limited to):
∘ memory (verbal, nonverbal, immediate, delayed)
∘ information processing speed
∘ attention
∘ executive function
∘ motor
∘ visual perception
∘ multi-tasking
∘ navigation
∘ intelligence
∘ working memory
∘ task switching
∘ response inhibition
∘ psychomotor speed
∘ verbal functions (fluency/naming/vocabulary)
∘ response time
∘ double decision
∘ sustained attention
∘ divided attention
∘ selective attention
∘ reasoning
∘ auditory processing

A memory score and/or information processing speed score are determined before and after treatment cycles by cognitive assessment tools (e.g. Neurotrax, BrainHQ or any other suitable assessment tool).

### Example 2: Cognitive training comparative protocol 2

Cognitive training is carried out during hyperbaric, rich oxygen environment exposure for the purpose of cognitive capabilities improvement and/or enhancement. The synergistic effect of cognitive training carried out during hyperbaric oxygen therapy (HBOT) is assessed as compared to cognitive training between HBOT sessions and without HBOT.

Subjects undergoing the treatment are patients with brain injury (stroke/TBI), age 40-55. Subjects' screening (inclusion criteria) - at least 1 standard deviation (SD) below average in at least one cognitive domain, as measured using a computerized cognitive assessment (e.g. Neurotrax, or any other suitable assessment tool).

Two groups of 5 patients each are tested according to the following grouping:
1. Group 1: HBOT with cognitive training intra-chamber (60 cycles, for example 1 cycle per day for 60 days)
2. Group 2 (control 1): HBOT with cognitive training outside the chamber (60 daily cycles)
3. Group 3 (control 2): Cognitive training tasks only (60 daily cycles), or both groups perform cognitive training for 40-60 sessions without HBOT prior to separation into Group 1 and Group 2.

### Treatment parameters:

- Pressure in chamber: 2 ATA
- Oxygen level: 100%
- Total time: 120 minutes per treatment cycle
- Oxygen net time: 90 minutes per cycle
- Administration of oxygen rich environment by mask
- Total number of cycles: 60
- Number of cycles per week: 5
- Decompression rate: 1 m/min
- Compression rate: 1 m/min
- Air breaks: 5 minutes air break (21% oxygen) between every 20 minutes of rich-oxygen environment administration
- Duration of cognitive training task: 20 minutes per cycle

### Exemplary cognitive training

o memory (verbal, nonverbal, immediate, delayed)
o information processing speed

A memory score and/or information processing speed score are determined before and after treatment cycles by BRAINHQ (every 5 cycles), and by Neurotrax (after 60 cycles), or by any other suitable assessment tool.

## Claims

1. A system (100) for use in a method of modifying cognitive performance of a subject that comprises treating said subject by one or more treatment cycles, each treatment cycle comprises exposing said subject to hyperbaric conditions and oxygen rich environment, and carrying out at least one cognitive training task during said exposure, the system comprising
at least one hyperbaric chamber (102) configured to enclose two or more subjects and apply hyperbaric conditions thereonto, the hyperbaric chamber comprises a plurality of stations (104), each of station configured to accommodate a subject to be treated simultaneously therein,
at least one controllable oxygen administration unit permitting forming an oxygen rich environment within the chamber and configured to administer said oxygen rich environment to said subject, wherein the at least one controllable oxygen administration unit is configured for administering said oxygen rich environment during defined time periods, with intervals of administration of lower oxygen levels in between said defined time periods during said treatment cycle,
said controllable oxygen administration unit comprises a plurality of oxygen supply means for providing the subject with said oxygen rich environment, the oxygen supplying means being connected via supply line (109) to a controllable oxygen source (108), permitting control of oxygen level, duration of exposure, and oxygen exposure profile/regimen for each of the stations (104) independently of one another,
the controllable oxygen administration unit further comprising an air source (110) is linked to each of the stations (104) via an air supply line (111), permitting exposure of the subjects to air atmosphere in between oxygen-rich atmosphere exposure periods,
said oxygen supply means being selected from a personal mask, a hood and a cannula, and
each station within the chamber comprising at least one means of cognitive training (106) configured to permit a subject to carry out at least one cognitive training task, said at least one means for cognitive training comprising a dedicated device or tool being configured for subject interaction therewith during said cognitive training.

2. The system of claim 1, further comprising at least one means of physical training within the chamber configured to permit the subject to carry out at least one physical training task.

3. The system of claim 1 or 2, further comprising at least one monitoring unit for monitoring at least one physical parameter of the subject.

4. The system of any one of claims 1 to 3, further comprising at least one controller for operating and controlling the system.

5. The system of any one of claims 1 to 4, wherein said at least one means of cognitive training is configured for permitting each of the subjects to independently carry out at least one cognitive training task.

6. The system of any one of the preceding claims, wherein said wherein said at least one means of cognitive training is configured for permitting each of the subjects to carry out a different cognitive training task.

7. The system of any one of claims 1 to 6, wherein the chamber further comprises at least one compression sub-chamber, at least one training sub-chamber, and at least one decompression sub-chamber, the compression sub-chamber, training sub-chamber and decompression sub-chamber being linked to one another as to permit the subject to migrate therebetween, optionally wherein the at least one compression sub-chamber, at least one training sub-chamber, and at least one decompression sub-chamber are integral one with the other.

8. The system of any one of the preceding claims, wherein treatment conditions are the same in each treatment cycle.

9. The system of any one of claims 1 to 7, wherein treatment conditions differ from treatment cycle to treatment cycle.

10. The system of any one of claims 1 to 9, further comprising means for assessing cognitive performance of the subject before said exposure to obtain a first score, assessing cognitive performance of said subject after said exposure to obtain a second score, and comparing said first score and second score to obtain an evaluation score of the subject.

11. The system of any one of claims 1 to 10, wherein said at least one cognitive training task is one or more tasks selected from verbal memory task, nonverbal memory task, immediate memory task, delayed memory task, information processing speed task, attention task, executive function task, motoric task, visual perception task, multi-tasking assignment, visual spatial task, verbal task, navigational task, intelligence task, working memory task, task switching, response inhibition task, psychomotor speed task, verbal functions task (fluency/naming/vocabulary), response time, double decision, sustained attention, divided attention, selective attention, working memory, long term memory, reasoning, auditory processing, and combinations thereof.

12. The system of any one of claims 1 to 11, wherein said at least one means for cognitive training comprising a dedicated device or tool being configured for subject interaction therewith during said cognitive training, and the system being configured to provide assessment of cognitive performance during said treatment cycle by providing an ongoing evaluation score for the subject during the treatment cycle.

## Patentansprüche

1. System (100) zur Verwendung in einem Verfahren zum Verändern einer kognitiven Leistung eines Individuums, das das Behandeln des Individuums mit einem oder mehreren Behandlungszyklen umfasst, wobei jeder Behandlungszyklus das Exponieren des Individuums gegenüber hyperbaren Bedingungen und sauerstoffreicher Umgebung und das Durchführen zumindest einer kognitiven Trainingsaufgabe während der Exposition umfasst, wobei das System umfasst:
zumindest eine Hyperbarkammer (102), die dazu konfiguriert ist, zwei oder mehr Individuen zu umschließen und diese gegenüber hyperbaren Bedingungen zu exponieren, wobei die Hyperbarkammer eine Mehrzahl von Stationen (104) umfasst, wobei jede Station dazu konfiguriert ist, ein Individuum aufzunehmen, das gleichzeitig darin zu behandeln ist,
zumindest eine steuerbare Sauerstoffverabreichungseinheit, die das Bilden einer sauerstoffreichen Umgebung innerhalb der Kammer ermöglicht und dazu konfiguriert ist, die sauerstoffreiche Umgebung an das Individuum zu verabreichen, wobei die zumindest eine steuerbare Sauerstoffverarbeichungseinheit dazu konfiguriert ist, die sauerstoffreiche Umgebung während definierter Zeiträume zu verabreichen, wobei Intervalle der Verabreichung geringer Sauerstoffmengen zwischen den definierten Zeiträumen während des Behandlungszyklus vorliegen,
die steuerbare Sauerstoffverabreichungseinheit eine Mehrzahl von Sauerstoffzufuhrmitteln zum Versorgen des Individuums mit der sauerstoffreichen Umgebung umfasst, wobei das Sauerstoffzufuhrmittel über eine Zufuhrleitung (109) mit einer steuerbaren Sauerstoffquelle (108) verbunden ist, die eine Steuerung einer Sauerstoffmenge, einer Expositionsdauer und eines Sauerstoffexpositionsprofils/-plans für jede der Stationen (104) unabhängig voneinander ermöglicht,
wobei die steuerbare Sauerstoffverabreichungseinheit ferner eine Luftquelle (110) umfasst, die mit jeder der Stationen (104) über eine Luftzufuhrleitung (111) verbunden ist, die eine Exposition der Individuen gegenüber einer Luftatmosphäre zwischen Zeiträumen der Exposition gegenüber einer sauerstoffreichen Atmosphäre ermöglicht,
wobei das Sauerstoffzufuhrmittel aus einer Gesichtsmaske, einer Haube und einer Kanüle ausgewählt ist,
und
wobei jede Station innerhalb der Kammer zumindest ein Mittel für kognitives Training (106) umfasst, das dazu konfiguriert ist, einem Individuum das Durchführen zumindest einer kognitiven Trainingsaufgabe zu ermöglichen, wobei das zumindest eine Mittel für kognitives Training eine dedizierte Vorrichtung oder ein dediziertes Werkzeug umfasst, die bzw. das dazu konfiguriert ist, während des kognitiven Trainings mit dem Individuum zu interagieren.

2. System nach Anspruch 1, das ferner zumindest ein Mittel für körperliches Training innerhalb der Kammer umfasst, das dazu konfiguriert ist, dem Individuum das Durchführen zumindest einer körperlichen Trainingsaufgabe zu ermöglichen.

3. System nach Anspruch 1 oder 2, das ferner zumindest eine Überwachungseinheit zum Überwachen zumindest eines körperlichen Parameters des Individuums umfasst.

4. System nach einem der Ansprüche 1 bis 3, das ferner zumindest eine Steuerung zum Betreiben und Steuern des Systems umfasst.

5. System nach einem der Ansprüche 1 bis 4, wobei das zumindest eine Mittel für kognitives Training dazu konfiguriert ist, jedem der Individuen das unabhängige Durchführen zumindest einer kognitiven Trainingsaufgabe zu ermöglichen.

6. System nach einem der vorstehenden Ansprüche, wobei das wobei das zumindest eine Mittel für kognitives Training dazu konfiguriert ist, jedem der Individuen das Durchführen einer unterschiedlichen kognitiven Trainingsaufgabe zu ermöglichen.

7. System nach einem der Ansprüche 1 bis 6, wobei die Kammer ferner zumindest eine Kompressionsteilkammer, zumindest eine Trainingsteilkammer und zumindest eine Dekompressionsteilkammer umfasst, wobei die Kompressionsteilkammer, die Trainingsteilkammer und die Dekompressionsteilkammer miteinander verbunden sind, um dem Individuum das Wechseln zwischen diesen zu ermöglichen, wahlweise wobei die zumindest eine Kompressionsteilkammer, die zumindest eine Trainingsteilkammer und die zumindest eine Dekompressionsteilkammer in Bezug aufeinander integral ausgebildet sind.

8. System nach einem der vorstehenden Ansprüche, wobei Behandlungsbedingungen in jedem Behandlungszyklus gleich sind.

9. System nach einem der Ansprüche 1 bis 7, wobei sich Behandlungsbedingungen von Behandlungszyklus zu Behandlungszyklus unterscheiden.

10. System nach einem der Ansprüche 1 bis 9, das ferner ein Mittel zum Bewerten einer kongnitiven Leistung des Individuums vor der Exposition unter Erhalt eines ersten Werts, zum Bewerten einer kognitiven Leistung des Individuums nach der Exposition unter Erhalt eines zweiten Werts und zum Vergleichen des ersten Werts und des zweiten Werts unter Erhalt eines Beurteilungswerts des Individuums umfasst.

11. System nach einem der Ansprüche 1 bis 10, wobei die zumindest eine kognitive Trainingsaufgabe eine oder mehrere Aufgaben ist, die aus verbaler Gedächtnisaufgabe, nonverbaler Gedächtnisaufgabe, unmittelbarer Gedächtnisaufgabe, verzögerter Gedächtnisaufgabe, Aufgabe zur Informationsverarbeitungsgeschwindigkeit, Aufmerksamkeitsaufgabe, Aufgabe zu exekutiver Funktion, motorischer Aufgabe, Aufgabe zur visuellen Wahrnehmung, Multitaskingaufgabe, visuell-räumlicher Aufgabe, verbaler Aufgabe, Navigationsaufgabe, Intelligenzaufgabe, Arbeitsgedächtnisaufgabe, Aufgabenwechsel, Reaktionshemmungsaufgabe, Aufgabe zur psychomotorischen Geschwindigkeit, Aufgabe zu verbalen Funktionen (Sprachfluss/Benennung/Vokabular), Reaktionszeit, Double Decision, aufrechterhaltener Aufmerksamkeit, geteilter Aufmerksamkeit, selektiver Aufmerksamkeit, Arbeitsgedächtnis, Langzeitgedächtnis, Argumentation, auditiver Verarbeitung und Kombinationen davon ausgewählt sind.

12. System nach einem der Ansprüche 1 bis 11, wobei das zumindest eine Mittel für kognitives Training eine dedizierte Vorrichtung oder ein dediziertes Werkzeug umfasst, die bzw. das dazu konfiguriert ist, während des kognitiven Trainings mit diesem zu interagieren, und wobei das System dazu konfiguriert ist, eine Bewertung einer kognitiven Leistung während des Behandlungszyklus vorzusehen, indem es während des Behandlungszyklus einen fortlaufenden Beurteilungswert für das Individuum bereitstellt.

## Revendications

1. Système (100) à utiliser dans un procédé de modification des performances cognitives d'un sujet, comprenant le traitement dudit sujet par un ou plusieurs cycles de traitement, chaque cycle de traitement comprenant l'exposition dudit sujet à des conditions hyperbares et à un environnement riche en oxygène, et la réalisation d'au moins une tâche d'entraînement cognitif pendant ladite exposition, le système comprenant
au moins une chambre hyperbare (102) configurée pour enfermer deux ou plusieurs sujets et leur appliquer des conditions hyperbares, la chambre hyperbare comprenant une pluralité de stations (104), chacune des stations étant configurée pour accueillir un sujet à traiter simultanément,
au moins une unité d'administration d'oxygène contrôlable permettant de former un environnement riche en oxygène dans la chambre et configurée pour administrer ledit environnement riche en oxygène audit sujet, dans lequel l'au moins une unité d'administration d'oxygène contrôlable est configurée pour administrer ledit environnement riche en oxygène pendant des périodes de temps définies, avec des intervalles d'administration de niveaux d'oxygène inférieurs entre lesdites périodes de temps définies pendant ledit cycle de traitement,
ladite unité d'administration d'oxygène contrôlable comprend une pluralité de moyens d'alimentation en oxygène pour fournir au sujet ledit environnement riche en oxygène, les moyens d'alimentation en oxygène étant connectés via une ligne d'alimentation (109) à une source d'oxygène contrôlable (108), permettant le contrôle du niveau d'oxygène, de la durée d'exposition et du profil/régime d'exposition à l'oxygène pour chacune des stations (104) indépendamment les unes des autres,
l'unité d'administration d'oxygène contrôlable comprenant en outre une source d'air (110) est reliée à chacune des stations (104) via une conduite d'alimentation en air (111), permettant l'exposition des sujets à l'atmosphère de l'air entre les périodes d'exposition à une atmosphère riche en oxygène,
lesdits moyens d'alimentation en oxygène étant choisis parmi un masque personnel, une tente et une canule, et
chaque station à l'intérieur de la chambre comprenant au moins un moyen d'entraînement cognitif (106) configuré pour permettre à un sujet d'effectuer au moins une tâche d'entraînement cognitif, ledit au moins un moyen d'entraînement cognitif comprenant un dispositif ou un outil dédié étant configuré pour une interaction du sujet avec celui-ci pendant ledit entraînement cognitif.

2. Système selon la revendication 1, comprenant en outre au moins un moyen d'entraînement physique à l'intérieur de la chambre configuré pour permettre au sujet d'effectuer au moins une tâche d'entraînement physique.

3. Système selon la revendication 1 ou 2, comprenant en outre au moins une unité de surveillance pour surveiller au moins un paramètre physique du sujet.

4. Système selon l'une quelconque des revendications 1 à 3, comprenant en outre au moins un contrôleur pour faire fonctionner et contrôler le système.

5. Système selon l'une quelconque des revendications 1 à 4, dans lequel ledit au moins un moyen d'entraînement cognitif est configuré pour permettre à chacun des sujets d'effectuer indépendamment au moins une tâche d'entraînement cognitif.

6. Système selon l'une quelconque des revendications précédentes, dans lequel ledit au moins un moyen d'entraînement cognitif est configuré pour permettre à chacun des sujets d'effectuer une tâche d'entraînement cognitif différente.

7. Système selon l'une quelconque des revendications 1 à 6, dans lequel la chambre comprend en outre au moins une sous-chambre de compression, au moins une sous-chambre d'entraînement et au moins une sous-chambre de décompression, la sous-chambre de compression, la sous-chambre d'entraînement et la sous-chambre de décompression étant reliées les unes aux autres de manière à permettre au sujet de migrer entre elles, éventuellement dans lequel l'au moins une sous-chambre de compression, l'au moins une sous-chambre d'entraînement et l'au moins une sous-chambre de décompression sont solidaires l'une de l'autre.

8. Système selon l'une quelconque des revendications précédentes, dans lequel les conditions de traitement sont les mêmes dans chaque cycle de traitement.

9. Système selon l'une quelconque des revendications 1 à 7, dans lequel les conditions de traitement diffèrent d'un cycle de traitement à l'autre.

10. Système selon l'une quelconque des revendications 1 à 9, comprenant en outre des moyens pour évaluer les performances cognitives du sujet avant ladite exposition pour obtenir un premier score, évaluer les performances cognitives dudit sujet après ladite exposition pour obtenir un second score, et comparer ledit premier score et ledit second score pour obtenir un score d'évaluation du sujet.

11. Système selon l'une quelconque des revendications 1 à 10, dans lequel ladite au moins une tâche d'entraînement cognitif est une ou plusieurs tâches sélectionnées parmi une tâche de mémoire verbale, une tâche de mémoire non verbale, une tâche de mémoire immédiate, une tâche de mémoire différée, une tâche de vitesse de traitement de l'information, une tâche d'attention, une tâche de fonction exécutive, une tâche motrice, une tâche de perception visuelle, une affectation multitâche, une tâche visuo-spatiale, une tâche verbale, une tâche de navigation, une tâche d'intelligence, une tâche de mémoire de travail, une commutation de tâches, une tâche d'inhibition de réponse, une tâche de vitesse psychomotrice, une tâche de fonctions verbales (fluidité/dénomination/vocabulaire), un temps de réponse, une double décision, une attention soutenue, une attention divisée, une attention sélective, une mémoire de travail, une mémoire à long terme, un raisonnement, un traitement auditif et des combinaisons de celles-ci.

12. Système selon l'une quelconque des revendications 1 à 11, dans lequel ledit au moins un moyen d'entraînement cognitif comprenant un dispositif ou un outil dédié étant configuré pour une interaction du sujet avec celui-ci pendant ledit entraînement cognitif, et le système étant configuré pour fournir une évaluation des performances cognitives pendant ledit cycle de traitement en fournissant un score d'évaluation continu pour le sujet pendant le cycle de traitement.
